(19) Europäisches Patentamt
European Patent Office
Office européen des brevets

(11) **EP 3 749 376 B1**

(12) **EUROPÄISCHE PATENTSCHRIFT**

(45) Veröffentlichungstag und Bekanntmachung des Hinweises auf die Patenterteilung:
**20.04.2022 Patentblatt 2022/16**

(21) Anmeldenummer: **19701863.3**

(22) Anmeldetag: **28.01.2019**

(51) Internationale Patentklassifikation (IPC):
**A61L 15/60** (2006.01)   **B65G 53/04** (2006.01)
**C08J 3/12** (2006.01)

(52) Gemeinsame Patentklassifikation (CPC):
**A61L 15/60; B65G 53/04; C08J 3/126**

(86) Internationale Anmeldenummer:
**PCT/EP2019/051966**

(87) Internationale Veröffentlichungsnummer:
**WO 2019/154652 (15.08.2019 Gazette 2019/33)**

(54) **VERFAHREN ZUR PNEUMATISCHEN FÖRDERUNG VON SUPERABSORBERPARTIKELN**

METHOD FOR THE PNEUMATIC DELIVERY OF SUPERABSORBENT PARTICLES

PROCÉDÉ POUR ASSURER LE TRANSPORT PNEUMATIQUE DE PARTICULES DE SUPERABSORBANTS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **06.02.2018 EP 18155317**

(43) Veröffentlichungstag der Anmeldung:
**16.12.2020 Patentblatt 2020/51**

(73) Patentinhaber: **BASF SE**
**67056 Ludwigshafen am Rhein (DE)**

(72) Erfinder:
• **BAUER, Stephan**
**67056 Ludwigshafen (DE)**
• **BAUMANN, Katrin**
**67056 Ludwigshafen (DE)**
• **TOENNESSEN, Markus**
**67056 Ludwigshafen (DE)**
• **DANIEL, Thomas**
**67056 Ludwigshafen (DE)**
• **WOLF, Hanno Ruediger**
**67056 Ludwigshafen (DE)**

(74) Vertreter: **BASF IP Association**
**BASF SE**
**GBI-C006**
**67056 Ludwigshafen (DE)**

(56) Entgegenhaltungen:
**EP-A2- 0 755 964    WO-A1-2006/069732**

**Beschreibung**

Die vorliegende Erfindung betrifft ein Verfahren zur pneumatischen Förderung von Superabsorberpartikeln, wobei die Superabsorberpartikel vor der pneumatischen Förderung mit einer wässrigen Wachsdispersion versetzt wurden, das Wachs eine Glasübergangstemperatur von mindestens 65°C aufweist und bezogen auf die unbehandelten Superabsorberpartikel von 0,020 bis 0,20 Gew.-% Wachs eingesetzt wurden.

Superabsorber werden zur Herstellung von Windeln, Tampons, Damenbinden und anderen Hygieneartikeln, aber auch als wasserzurückhaltende Mittel im landwirtschaftlichen Gartenbau verwendet. Die Superabsorber werden auch als wasserabsorbierende Polymere bezeichnet.

Die Herstellung von Superabsorbern wird in der Monographie "Modern Superabsorbent Polymer Technology", F.L. Buchholz und A.T. Graham, Wiley-VCH, 1998, Seiten 71 bis 103, beschrieben.

Die Beschichtung von Superabsorbern mit Wachsen wird beispielsweise in EP 0 755 964 A2 und WO 2008/077779 A1 beschrieben.

Aufgabe der vorliegenden Erfindung war die Bereitstellung verbesserter Superabsorber, insbesondere von Superabsorbern mit verbesserten Transporteigenschaften bei der pneumatischen Förderung.

Gelöst wurde die Aufgabe durch ein Verfahren zur pneumatischen Förderung von Superabsorberpartikeln, dadurch gekennzeichnet, dass die Superabsorberpartikel vor der pneumatischen Förderung mit einer wässrigen Wachsdispersion versetzt wurden, das Wachs eine Glasübergangstemperatur von mindestens 65°C aufweist und bezogen auf die unbehandelten Superabsorberpartikel von 0,020 bis 0,20 Gew.-% Wachs eingesetzt wurden.

Grundsätzlich lassen sich bei der pneumatischen Förderung drei verschiedene Förderarten unterscheiden:

- Bei Flugförderung und Strömförderung im Bereich hoher Gasgeschwindigkeiten gelten annähernd die Gesetze des frei angeströmten Einzelkorns. Es ist die klassische Art der pneumatischen Förderung. Es treten keinerlei Produktablagerungen auf. Es liegt eine im Wesentlichen gleichmäßige Fördergutverteilung in der Förderleitung vor.
- Sinkt die Gasgeschwindigkeit, dann kommt man in den Bereich der Strähnenförderung, wo das Fördergut vor allem in der unteren Hälfte der Förderleitung strömt. In der oberen Hälfte der Förderleitung liegt Flugförderung vor.
- Bei kleinen Gasgeschwindigkeiten erfolgt die Förderung äußerst schonend als Dichtstromförderung (Pfropfenförderung, Impulsförderung) mit hohem Druckverlust.

Prinzipiell kann die Druckförderung mit langsameren Fördergeschwindigkeiten arbeiten als die Saugförderung, da im Überdruck die Druckreserven größer sind als im Vakuum, und da mit steigendem Druck die Fördergasdichte, die das Produkt vorantreibt, zunimmt.

Da Fördergas kompressibel ist, herrscht in der Förderleitung kein konstanter Druck, sondern am Anfang ein höherer als am Ende. Dadurch verändert sich aber auch das Gasvolumen, so dass am Anfang, bei höherem Druck, langsamere Gasgeschwindigkeiten vorherrschen und am Ende, bei niedrigerem Druck, höhere Gasgeschwindigkeiten.

Zu niedrige Fördergeschwindigkeiten im Bereich der Strähnenförderung sind problematisch, da in dem instabilen Bereich zwischen der Dichtstromförderung und der Strähnenförderung keine stabile Förderung möglich ist. Vielmehr können die dabei auftretenden mechanischen Belastungen zu schweren Schäden am Fördersystem, bis zum Herausreißen der Förderleitungen aus den Halterungen, führen.

Die optimale Gasanfangsgeschwindigkeit bei der pneumatischen Förderung hängt vom Durchmesser der Förderleitung ab. Diese Abhängigkeit wird am besten mit der Froude-Zahl beschrieben:

$$Fr = \frac{v}{\sqrt{D \times g}}$$

Fr   Froude-Zahl
v    Gasgeschwindigkeit
D    Innendurchmesser der Transportleitung
g    Erdbeschleunigung

Die Gasanfangsgeschwindigkeit bei der pneumatischen Förderung entspricht vorzugsweise einer Froude-Zahl von 2 bis 40, ganz besonders bevorzugt von 5 bis 30, ganz besonders bevorzugt von 10 bis 20. Die Fördergutbeladung bei der pneumatischen Förderung beträgt vorzugsweise von 1 bis 30 kg/kg, besonders bevorzugt von 5 bis 25 kg/kg, ganz besonders bevorzugt von 10 bis 20 kg/kg, wobei die Fördergutbeladung der Quotient aus Fördergutmassenstrom und Gasmassenstrom ist.

Die Glasübergangstemperatur des Wachses beträgt vorzugsweise mindestens 70°C, besonders bevorzugt mindestens 75°C, ganz besonders bevorzugt mindestens 80°C.

Mit der Glasübergangstemperatur Tg, ist der Grenzwert der Glasübergangstemperatur gemeint, dem diese gemäß G. Kanig (Kolloid-Zeitschrift & Zeitschrift für Polymere, Band 190, Seite 1, Gleichung 1) mit zunehmendem Molekulargewicht zustrebt. Die Tg wird nach dem DSC-Verfahren gemäß DIN ISO 11357-2:2014-07 ermittelt (Differential Scanning Calorimetry, 20 K/min, midpoint-Messung). Die Tg-Werte für die Homopolymerisate der meisten Monomeren sind bekannt und z.B. in Ullmann's Ecyclopedia of Industrial Chemistry, VCH Weinheim, 1992, Ed. 5, Vol. A21, Seiten 169ff, aufgeführt; weitere Quellen für Glasübergangstemperaturen von Homopolymerisaten bilden z.B. J. Brandrup, E.H. Immergut, Polymer Handbook, Ed. 15, J. Wiley, New York 1996, Ed. 2, J.Wiley, New York 1975, und Ed. 3, J. Wiley, New York 1989. Für die Erzielung der gewünschten Glasübergangstemperatur Tg des Wachses durch Auswahl geeigneter Arten und Mengen von Monomeren ist die Formel von Fox (T.G. Fox, Bull. Amer. Phys. Soc. (Ser. II) Jahrgang 1956, Band 1, Seiten 123ff) hilfreich, nach der für die Glasübergangstemperatur von Copolymerisaten in guter Näherung gilt:

$$1/Tg = x_1/Tg_1 + X_2/Tg_2 + \ldots\ldots + x_n/Tg_n$$

worin $x_1$, $x_2$, ... $x_n$ die Massenbrüche der Monomeren und $Tg_1$, $Tg_2$, ..., $Tg_n$ die Glasübergangstemperatur der jeweils aus einem der Monomeren 1, 2, ..., n aufgebauten Homopolymerisates in Kelvin bedeuten.

Die Einsatzmenge an Wachs beträgt vorzugsweise von 0,025 bis 0,15 Gew.-%, besonders bevorzugt von 0,030 bis 0,1 Gew.-%, ganz besonders bevorzugt von 0,035 bis 0,08 Gew.-%, jeweils bezogen auf die unbehandelten Superabsorberpartikel.

In einer bevorzugten Ausführungsform der vorliegenden Erfindung ist das Wachs ein Copolymer aus 70 bis 95 mol-% mindestens einem ethylenisch ungesättigten Kohlenwasserstoff und 5 bis 30 mol-% mindestens einer ethylenisch ungesättigten Carbonsäure. Ganz besonders bevorzugt sind Ethylen-Acrylsäure-Copolymere und Ethylen-Methacrylsäure-Copolymere Beispiele für derartige Copolymere sind Luwax® EAS 5 (BASF SE; Ludwigshafen; Deutschland), Nucrel® 960 (DuPont Company; Wilmington; Vereinigte Staaten von Amerika) und Primacor® 5980I (The Dow Chemical Company; Midland; Vereinigte Staaten von Amerika).

Bevorzugt werden die Wachse als wässrige Dispersionen eingesetzt mit einem Feststoffgehalt von vorzugsweise kleiner 50 Gew.-%, bevorzugt kleiner 40 Gew.-% und ganz bevorzugt kleiner 30% Gew.-%. Ganz bevorzugt sind Wachsdispersionen die nur aus Wasser, Wachs und Alkalihydroxid als Dispergierhilfsmittel bestehen.

Derartige Wachsdispersionen werden üblicherweise durch dispergieren einer Wachsschmelze in Wasser hergestellt und mit geeigneten Dispergierhilfsmitteln stabilisiert.

Beispiele für derartige Wachsdispersionen sind Poligen® WE 1, Poligen® WE 4, Poligen® CE 18 (jeweils BASF SE; Ludwigshafen; Deutschland), Cohesa® 001 (Honeywell International; Morris Plains; Vereinigte Staaten von Amerika), Michem® Emulsion 34935 (Michelman, Inc.; Cincinanati; Vereinigte Staaten von Amerika).

In einer ganz bevorzugten Ausführungsform der vorliegenden Erfindung werden wässrige Wachsdispersionen bestehend aus Ethylen-Acrylsäure-Copolymer und Ethylen-Methacrylsäure-Copolymer eingesetzt, die anschließend nur mit Natrium oder Kaliumhydroxid als Dispergierhilfsmittel stabilisiert sind.

Der pH-Wert der Wachsdispersion beträgt vorzugsweise mindestens 7, ganz bevorzugt mindestens 8 und ganz besonders bevorzugt mindestens 9.

Die Superabsorberpartikel weisen eine mittlere Partikelgröße von vorzugsweise 150 bis 850 $\mu$m, besonders bevorzugt 200 bis 600 $\mu$m, ganz besonders bevorzugt von 250 bis 500 $\mu$m, auf.

Die Superabsorberpartikel weisen in einer bevorzugten Ausführungsform der vorliegenden Erfindung eine mittlere Sphärizität (mSPHT) von vorzugsweise größer 0,72, besonders bevorzugt von größer 0,76, ganz besonders bevorzugt von größer 0,80, auf.

Die Temperatur der Superabsorberpartikel während der pneumatischen Förderung beträgt vorzugsweise mindestens 20°C, besonders bevorzugt mindestens 30°C, ganz besonders bevorzugt mindestens 40°C, und vorzugsweise mindestens 20°C weniger, besonders bevorzugt mindestens 25°C weniger, ganz besonders bevorzugt mindestens 30°C weniger, als die Glasübergangstemperatur des Wachses.

Der vorliegenden Erfindung liegt die Erkenntnis zugrunde, dass sich Transporteigenschaften der Superabsorberpartikel durch Behandeln mit einer wässrigen Wachsdispersion verbessern lassen. Mit Wachs beschichtete Superabsorberpartikel verursachen bei der pneumatischen Förderung einen niedrigeren Differenzdruck, wodurch die Kapazität der Förderleitung erhöht wird. Damit die Absorptionseigenschaften der Superabsorberpartikel nicht negativ beeinflusst werden, darf die Menge an Wachs nicht zu hoch sein und darf das Wachs keinen geschlossenen Film auf der Partikeloberfläche bilden. Letzteres wird durch eine ausreichend hohe Glasübergangstemperatur gewährleistet.

Im Folgenden wird die Herstellung der Superabsorber näher erläutert:
Die Superabsorber können durch Polymerisation einer Monomerlösung oder -suspension, enthaltend

     a) mindestens ein ethylenisch ungesättigtes, säuregruppentragendes Monomer, das zumindest teilweise neutralisiert sein kann,
b) mindestens einen Vernetzer und
c) mindestens einen Initiator,

hergestellt werden und sind üblicherweise wasserunlöslich.

Die Monomeren a) sind vorzugsweise wasserlöslich, d.h. die Löslichkeit in Wasser bei 23°C beträgt typischerweise mindestens 1 g/100 g Wasser, vorzugsweise mindestens 5 g/100 g Wasser, besonders bevorzugt mindestens 25 g/100 g Wasser, ganz besonders bevorzugt mindestens 35 g/100 g Wasser.

Geeignete Monomere a) sind beispielsweise ethylenisch ungesättigte Carbonsäuren, wie Acrylsäure, Methacrylsäure, und Itaconsäure. Besonders bevorzugte Monomere sind Acrylsäure und Methacrylsäure. Ganz besonders bevorzugt ist Acrylsäure.

Der Anteil an Acrylsäure und/oder deren Salzen an der Gesamtmenge der Monomeren a) beträgt vorzugsweise mindestens 50 mol-%, besonders bevorzugt mindestens 90 mol-%, ganz besonders bevorzugt mindestens 95 mol-%.

Die Monomere a) enthalten üblicherweise Polymerisationsinhibitoren, vorzugsweise Hydrochinonmonomethylether (MEHQ), als Lagerstabilisator.

Die Monomerlösung enthält vorzugsweise bis zu 250 Gew.-ppm, bevorzugt höchstens 130 Gew.-ppm, besonders bevorzugt höchstens 70 Gew.-ppm, bevorzugt mindestens 10 Gew.- ppm, besonders bevorzugt mindestens 30 Gew.-ppm, insbesondere um 50 Gew.-ppm, Hydrochinonmonomethylether (MEHQ), jeweils bezogen auf das unneutralisierte Monomer a). Beispielsweise kann zur Herstellung der Monomerlösung ein ethylenisch ungesättigtes, säuregruppentragendes Monomer mit einem entsprechenden Gehalt an Hydrochinonmonomethylether (MEHQ) verwendet werden.

Geeignete Vernetzer b) sind Verbindungen mit mindestens zwei zur Vernetzung geeigneten Gruppen. Derartige Gruppen sind beispielsweise ethylenisch ungesättigte Gruppen, die in die Polymerkette radikalisch einpolymerisiert werden können, und funktionelle Gruppen, die mit den Säuregruppen des Monomers a) kovalente Bindungen ausbilden können. Weiterhin sind auch polyvalente Metallsalze, die mit mindestens zwei Säuregruppen des Monomeren a) koordinative Bindungen ausbilden können, als Vernetzer b) geeignet. Weitere geeignete Vernetzer b) sind die in US 2017/361305 beschriebenen "nano-clays", die in WO 2000/31157 A1 beschrieben Wassergläser und die in WO 99/55767 A1 beschriebenen Aluminate.

Vernetzer b) sind vorzugsweise Verbindungen mit mindestens zwei polymerisierbaren Gruppen, die in das Polymernetzwerk radikalisch einpolymerisiert werden können. Geeignete Vernetzer b) sind beispielsweise Ethylenglykoldimethacrylat, Diethylenglykoldiacrylat, Polyethylenglykoldiacrylat, Allylmethacrylat, Trimethylolpropantriacrylat, Triallylamin, Tetraallylammoniumchlorid, Tetraallyloxyethan, wie in EP 0 530 438 A1 beschrieben, Di- und Triacrylate, wie in EP 0 547 847 A1, EP 0 559 476 A1, EP 0 632 068 A1, WO 93/21237 A1, WO 03/104299 A1, WO 03/104300 A1, WO 03/104301 A1 und DE 103 31 450 A1 beschrieben, gemischte Acrylate, die neben Acrylatgruppen weitere ethylenisch ungesättigte Gruppen enthalten, wie in DE 103 31 456 A1 und DE 103 55 401 A1 beschrieben, oder Vernetzermischungen, wie beispielsweise in DE 195 43 368 A1, DE 196 46 484 A1, WO 90/15830 A1 und WO 02/032962 A2 beschrieben.

Bevorzugte Vernetzer b) sind Pentaerythrittriallylether, Tetraallyloxyethan, Methylenbismethacrylamid, 15-fach ethoxyliertes Trimethylolpropantriacrylat, Polyethylenglykoldiacrylat, Trimethylolpropantriacrylat und Triallylamin.

Die Menge an Vernetzer b) beträgt vorzugsweise 0,25 bis 1,5 Gew.-%, besonders bevorzugt 0,3 bis 1,2 Gew.-%, ganz besonders bevorzugt 0,4 bis 0,8 Gew.-%, jeweils berechnet auf die Gesamtmenge an eingesetztem Monomer a). Mit steigendem Vernetzergehalt sinkt die Zentrifugenretentionskapazität (CRC) und die Absorption unter einem Druck von 21,0 g/cm$^2$ durchläuft ein Maximum.

Als Initiatoren c) können sämtliche unter den Polymerisationsbedingungen Radikale erzeugende Verbindungen eingesetzt werden, beispielsweise thermische Initiatoren, Redox-Initiatoren, Photoinitiatoren. Geeignete Redox-Initiatoren sind Natriumperoxodisulfat/Ascorbinsäure, Wasserstoffperoxid/Ascorbinsäure, Natriumperoxodisulfat/Natriumbisulfit und Wasserstoffperoxid/Natriumbisulfit. Vorzugsweise werden Mischungen aus thermischen Initiatoren und Redox-Initiatoren eingesetzt, wie Natriumperoxodisulfat/Wasserstoffperoxid/Ascorbinsäure. Als reduzierende Komponente wird vorzugsweise das Dinatriumsalz der 2-Hydroxy-2-sulfonatoessigsäure oder ein Gemisch aus dem Natriumsalz der 2-Hydroxy-2-sulfinatoessigsäure, dem Dinatriumsalz der 2-Hydroxy-2-sulfonatoessigsäure und Natriumbisulfit eingesetzt. Derartige Gemische sind als Brüggolite® FF6 und Brüggolite® FF7 (Brüggemann Chemicals; Heilbronn; Deutschland) erhältlich. Es kann aber auch die reine 2-Hydroxy-2-sulfonatoessigsäure oder ein Salz davon als reduzierende Komponente eingesetzt werden, insbesondere wenn Ascorbinsäure mitverwendet wird.

In die Monomerlösung können vor oder während der Polymerisation noch Chelatbildner und 2-Hydroxycarbonsäuren zugesetzt werden wie beispielsweise in der WO 2017/170604 A1 beschrieben.

Üblicherweise wird eine wässrige Monomerlösung verwendet. Der Wassergehalt der Monomerlösung beträgt vorzugsweise von 40 bis 75 Gew.-%, besonders bevorzugt von 45 bis 70 Gew.- %, ganz besonders bevorzugt von 50 bis 65 Gew.-%. Es ist auch möglich Monomersuspensionen, d.h. Monomerlösungen mit die Löslichkeit überschreitendem Monomer a), beispielsweise Natriumacrylat, einzusetzen. Mit steigendem Wassergehalt steigt der Energieaufwand bei der anschließenden Trocknung und mit sinkendem Wassergehalt kann die Polymerisationswärme nur noch ungenügend abgeführt werden.

Die Säuregruppen der erhaltenen Polymergele sind üblicherweise teilweise neutralisiert. Die Neutralisation wird auf der Stufe der Monomeren durchgeführt. Dies geschieht üblicherweise durch Einmischung des Neutralisationsmittels als wässrige Lösung oder bevorzugt auch als Feststoff. Der Neutralisationsgrad beträgt vorzugsweise von 25 bis 85 mol-%, besonders bevorzugt von 30 bis 80 mol-%, ganz besonders bevorzugt von 40 bis 75 mol-%, wobei die üblichen Neutralisationsmittel verwendet werden können, vorzugsweise Alkalimetallhydroxide, Alkalimetalloxide, Alkalimetallkarbonate oder Alkalimetallhydrogenkarbonate sowie deren Mischungen.

Statt Alkalimetallsalzen können auch Ammoniumsalze verwendet werden. Natrium und Kalium sind als Alkalimetalle besonders bevorzugt, ganz besonders bevorzugt sind jedoch Natriumhydroxid, Natriumkarbonat oder Natriumhydrogenkarbonat sowie deren Mischungen.

Im Folgenden wird die Lösungspolymerisation erläutert:

Geeignete Reaktoren für die Lösungspolymerisation sind beispielsweise Knetreaktoren oder Bandreaktoren. Im Kneter wird das bei der Polymerisation einer wässrigen Monomerlösung oder -suspension entstehende Polymergel durch beispielsweise gegenläufige Rührwellen kontinuierlich zerkleinert, wie in WO 2001/038402 A1 beschrieben. Die Polymerisation auf dem Band wird beispielsweise in DE 38 25 366 A1 und US 6,241,928 beschrieben. Bei der Polymerisation in einem Bandreaktor entsteht ein Polymergel, das zerkleinert werden muss, beispielsweise in einem Extruder oder Kneter.

Zur Verbesserung der Trocknungseigenschaften kann das mittels eines Kneters erhaltene zerkleinerte Polymergel zusätzlich extrudiert werden.

Das Polymergel wird dann in üblicherweise mit einem Umluftbandtrockner getrocknet bis der Restfeuchtegehalt vorzugsweise 0,5 bis 10 Gew.-%, besonders bevorzugt 1 bis 6 Gew.-%, ganz besonders bevorzugt 1,5 bis 4 Gew.-%, beträgt, wobei der Restfeuchtegehalt gemäß der von der EDANA empfohlenen Testmethode Nr. WSP 230.2-05 "Mass Loss Upon Heating" bestimmt wird. Bei einer zu hohen Restfeuchte weist das getrocknete Polymergel eine zu niedrige Glasübergangstemperatur $T_g$ auf und ist nur schwierig weiter zu verarbeiten. Bei einer zu niedrigen Restfeuchte ist das getrocknete Polymergel zu spröde und in den anschließenden Zerkleinerungsschritten fallen unerwünscht große Mengen an Polymerpartikeln mit zu niedriger Partikelgröße ("fines") an. Der Feststoffgehalt des Polymergels beträgt vor der Trocknung vorzugsweise von 25 und 90 Gew.-%, besonders bevorzugt von 35 bis 70 Gew.-%, ganz besonders bevorzugt von 40 bis 60 Gew.-%. Anschließend wird das getrocknete Polymergel gebrochen und optional grob zerkleinert.

Das getrocknete Polymergel wird hiernach üblicherweise gemahlen und klassiert, wobei zur Mahlung üblicherweise ein- oder mehrstufige Walzenstühle, bevorzugt zwei- oder dreistufige Walzenstühle, Stiftmühlen, Hammermühlen oder Schwingmühlen, eingesetzt werden können.

Die mittlere Partikelgröße der als Produktfraktion abgetrennten Polymerpartikel beträgt vorzugsweise von 150 bis 850 $\mu$m, besonders bevorzugt von 250 bis 600 $\mu$m, ganz besonders von 300 bis 500 $\mu$m. Die mittlere Partikelgröße der Produktfraktion kann mittels der von der EDANA empfohlenen Testmethode Nr. WSP 220.2-05 "Partikel Size Distribution" ermittelt werden, wobei die Massenanteile der Siebfraktionen kumuliert aufgetragen werden und die mittlere Partikelgröße graphisch bestimmt wird. Die mittlere Partikelgröße ist hierbei der Wert der Maschenweite, der sich für kumulierte 50 Gew.-% ergibt.

Der Anteil an Polymerpartikeln mit einer Partikelgröße von größer 150 $\mu$m beträgt vorzugsweise mindestens 90 Gew.-%, besonders bevorzugt mindestens 95 Gew.-%, ganz besonders bevorzugt mindestens 98 Gew.-%.

Polymerpartikel mit zu niedriger Partikelgröße senken die Gelbettpermeabilität (GBP). Daher sollte der Anteil zu kleiner Polymerpartikel ("fines") niedrig sein.

Zu kleine Polymerpartikel werden daher üblicherweise abgetrennt und in das Verfahren rückgeführt, vorzugsweise vor, während oder unmittelbar nach der Polymerisation, d.h. vor der Trocknung des Polymergels. Die zu kleinen Polymerpartikel können vor oder während der Rückführung mit Wasser und/oder wässrigem Tensid angefeuchtet werden.

Es ist auch möglich in späteren Verfahrensschritten zu kleine Polymerpartikel abzutrennen, beispielsweise nach der Oberflächennachvernetzung oder einem anderen Beschichtungsschritt. In diesem Fall sind die rückgeführten zu kleinen Polymerpartikel oberflächennachvernetzt bzw. anderweitig beschichtet, beispielsweise mit pyrogener Kieselsäure.

Wird zur Polymerisation ein Knetreaktor verwendet, so werden die zu kleinen Polymerpartikel vorzugsweise während des letzten Drittels der Polymerisation zugesetzt. Es ist aber auch möglich die zu kleinen Polymerpartikel in einem dem Polymerisationsreaktor nachgeschalteten Kneter oder Extruder in das Polymergel einzuarbeiten.

Werden die zu kleinen Polymerpartikel sehr früh zugesetzt, beispielsweise bereits zur Monomerlösung, so wird dadurch die Zentrifugenretentionskapazität (CRC) der erhaltenen Polymerpartikel gesenkt. Dies kann aber beispielsweise durch Anpassung der Einsatzmenge an Vernetzer b) kompensiert werden.

Der Anteil an Polymerpartikeln mit einer Partikelgröße von höchstens 850 $\mu$m, beträgt vorzugsweise mindestens 90 Gew.-%, besonders bevorzugt mindestens 95 Gew.-%, ganz besonders bevorzugt mindestens 98 Gew.-%.

Der Anteil an Polymerpartikeln mit einer Partikelgröße von höchstens 600 µm, beträgt vorzugsweise mindestens 90 Gew.-%, besonders bevorzugt mindestens 95 Gew.-%, ganz besonders bevorzugt mindestens 98 Gew.-%.

Polymerpartikel mit zu großer Partikelgröße senken die Quellgeschwindigkeit. Daher sollte der Anteil zu großer Polymerpartikel ebenfalls niedrig sein. Zu große Polymerpartikel werden daher üblicherweise abgetrennt und in die Mahlung rückgeführt.

Im Folgenden wird die Vertropfungspolymerisation erläutert:

Bei der Vertropfungspolymerisation wird die Monomerlösung mittels mindestens einer Bohrung unter Ausbildung von Tropfen in den Reaktor dosiert. Die Vertropfungspolymerisation wird beispielsweise in WO 2014/079694 A1 und WO 2015/110321 A1 beschrieben.

Die Bohrungen können sich beispielsweise in einer Vertropferplatte befinden. Die Anzahl und die Größe der Bohrungen werden gemäß der gewünschten Kapazität und Tropfengröße ausgewählt. Der Tropfendurchmesser beträgt dabei üblicherweise das 1,9-fache des Durchmessers der Bohrung. Wichtig ist hierbei, dass die zu vertropfende Flüssigkeit nicht zu schnell durch die Bohrung tritt bzw. der Druckverlust über die Bohrung nicht zu groß ist. Ansonsten wird die Flüssigkeit nicht vertropft, sondern der Flüssigkeitsstrahl wird infolge der hohen kinetischen Energie zerrissen (versprüht). Die Reynoldszahl bezogen auf den Durchsatz pro Bohrung und den Bohrungsdurchmesser ist vorzugsweise kleiner als 2.000, bevorzugt kleiner 1.600, besonders bevorzugt kleiner 1.400, ganz besonders bevorzugt kleiner 1.200.

Die Vertropferplatte weist vorzugsweise mindestens 5, besonders bevorzugt mindestens 25, ganz besonders bevorzugt mindestens 50, und vorzugsweise bis zu 750, besonders bevorzugt bis zu 500, ganz besonders bevorzugt bis zu 250, Bohrungen auf. Der Durchmesser der Bohrungen wird entsprechend der gewünschten Tropfengröße ausgewählt.

Der Durchmesser der Bohrungen beträgt vorzugsweise von 50 bis 500 µm, besonders bevorzugt von 100 bis 300 µm, ganz besonders bevorzugt von 150 bis 250 µm. Der Abstand der Bohrungen beträgt vorzugsweise 10 bis 50 mm, besonders bevorzugt 12 bis 40 mm, ganz besonders bevorzugt 15 bis 30 mm. Zu geringe Abstände führen zur Bildung von Agglomeraten.

Die Temperatur der Monomerlösung beim Durchtritt durch die Bohrungen beträgt vorzugsweise von 5 bis 80°C, besonders bevorzugt von 10 bis 70°C, ganz besonders bevorzugt von 30 bis 60°C.

Der Reaktor wird von einem Trägergas durchströmt. Dabei kann das Trägergas im Gleichstrom oder im Gegenstrom zu den frei fallenden Tropfen der Monomerlösung durch den Reaktor geführt werden, bevorzugt im Gleichstrom, d.h. von unten nach oben. Vorzugsweise wird das Trägergas nach einem Durchgang zumindest teilweise, bevorzugt zu mindestens 50%, besonders bevorzugt zu mindestens 75%, als Kreisgas in den Reaktor zurückgeführt. Üblicherweise wird eine Teilmenge des Trägergases nach jedem Durchgang ausgeschleust, vorzugsweise bis zu 10%, besonders bevorzugt bis zu 3%, ganz besonders bevorzugt bis zu 1%.

Der Sauerstoffgehalt des Trägergases beträgt vorzugsweise von 0,5 bis 15 Vol.-%, besonders bevorzugt von 1 bis 10 Vol.-%, ganz besonders bevorzugt von 2 bis 7 Vol.-%.

Das Trägergas enthält neben Sauerstoff vorzugsweise Stickstoff. Der Stickstoffgehalt des Trägergases beträgt vorzugsweise mindestens 80 Vol.-%, besonders bevorzugt mindesten 90 Vol.- %, ganz besonders bevorzugt mindestens 95 Vol.-%. Es können auch Gasmischungen verwendet werden. Das Trägergas kann auch mit Wasserdampf und/oder Acrylsäuredämpfen beladen werden.

Die Gasgeschwindigkeit wird vorzugsweise so eingestellt, dass die Strömung im Reaktor gerichtet ist, beispielsweise liegen keine der allgemeinen Strömungsrichtung entgegen gesetzte Konvektionswirbel vor, und beträgt üblicherweise 0,1 bis 2,5 m/s, vorzugsweise 0,3 bis 1,5 m/s bevorzugt von 0,5 bis 1,2 m/s, besonders bevorzugt 0,6 bis 1,0 m/s, ganz besonders bevorzugt 0,7 bis 0,9 m/s.

Das den Reaktor durchströmende Trägergas wird zweckmäßigerweise vor dem Reaktor auf die Reaktionstemperatur vorgewärmt.

Vorteilhaft wird die Gaseintrittstemperatur so geregelt, dass die Gasaustrittstemperatur, d.h. die Temperatur mit der das Trägergas den Reaktor verlässt üblicherweise von 90 bis 150°C, vorzugsweise von 100 bis 140°C, bevorzugt von 105 bis 135°C, besonders bevorzugt von 110 bis 130°C, ganz besonders bevorzugt von 115 bis 125°C, beträgt.

Die Reaktion kann im Überdruck oder im Unterdruck durchgeführt werden, ein Unterdruck von bis zu 100 mbar gegenüber dem Umgebungsdruck ist bevorzugt.

Das Reaktionsabgas, d.h. das den Reaktor verlassende Gas, kann beispielsweise in einem Wärmeaustauscher abgekühlt werden. Dabei kondensieren Wasser und nicht umgesetztes Monomer a). Danach kann das Reaktionsabgas zumindest teilweise wieder aufgewärmt und als Kreisgas in den Reaktor zurückgeführt werden. Ein Teil des Reaktionsabgases kann ausgeschleust und durch frisches Trägergas ersetzt werden, wobei im Reaktionsabgas enthaltenes Wasser und nicht umgesetzte Monomere a) abgetrennt und rückgeführt werden können.

Besonders bevorzugt ist ein Wärmeverbund, d.h., ein Teil der Abwärme beim Abkühlen des Abgases wird zum Aufwärmen des Kreisgases verwendet.

Die Reaktoren können begleitbeheizt werden. Die Begleitheizung wird dabei so eingestellt, dass die Wandtemperatur mindestens 5°C oberhalb der Reaktorinnentemperatur liegt und die Kondensation an den Reaktorwänden zuverlässig vermieden wird.

Im Folgenden wird die umgekehrte Suspensionspolymerisation beschrieben:

Bei der umgekehrten Suspensionspolymerisation wird die Monomerlösung während der Polymerisation in einem hydrophoben Lösungsmittel suspendiert. Die umgekehrte Suspensionspolymerisation wird beispielsweise in WO 2008/068208 A1 und WO 2015/062883 A2 beschrieben.

Als hydrophobe Lösungsmittel sind alle dem Fachmann zum Einsatz bei der Suspensionspolymerisation bekannten Lösungsmittel einsetzbar. Bevorzugt werden aliphatische Kohlenwasserstoffe, wie n-Hexan, n-Heptan, n-Oktan, n-Nonan, n-Dekan, Cyclohexan oder Mischungen daraus, verwendet. Hydrophobe Lösungsmittel weisen bei 23°C eine Löslichkeit in Wasser zu weniger als 5 g/100 g, vorzugsweise weniger als 1 g/100 g, besonders bevorzugt weniger als 0,5 g/100 g, auf.

Das hydrophobe Lösungsmittel siedet im Bereich von vorzugsweise 50 bis 150°C, besonders bevorzugt 60 bis 120°C, ganz besonders bevorzugt 70 bis 90°C.

Das Verhältnis zwischen hydrophoben Lösungsmittel und Monomerlösung beträgt 0,5 bis 3, bevorzugt 0,7 bis 2,5 und ganz bevorzugt von 0,8 bis 2,2.

Der mittlere Durchmesser der Monomerlösungstropfen in der Suspension beträgt, wenn keine Agglomeration durchgeführt wird, vorzugsweise mindestens 100 $\mu$m, besonders bevorzugt von 100 bis 1000 $\mu$m, besonders bevorzugt von 150 bis 850 $\mu$m, ganz besonders bevorzugt von 300 bis 600 $\mu$m, wobei der Tropfendurchmesser durch Lichtstreuung bestimmt werden kann und den volumengemittelten mittleren Durchmesser bedeutet.

Der Durchmesser der Monomerlösungstropfen kann über die eingetragene Rührenergie und durch geeignete Dispergierhilfsmittel eingestellt werden.

Zur Dispergierung der wässrigen Monomerlösung im hydrophoben Lösungsmittel bzw. zur Dispergierung der entstehenden Superabsorberpartikel werden vorzugsweise Dispergierhilfsmittel zugesetzt. Es kann sich dabei um anionische, kationische, nichtionische oder amphotere Tenside, oder natürliche, halbsynthetische oder synthetische Polymere handeln.

Anionische Tenside sind beispielsweise Natriumpolyoxyethylendodecylethersulfat und Natriumdodecylethersulfat. Ein kationisches Tensid ist beispielsweise Trimethylstearylammoniumchlorid. Ein amphoteres Tensid ist beispielsweise Carboxymethyldimethylcetylammonium. Nichtionische Tenside sind beispielsweise Saccharosefettsäureester, wie Saccharosemonostearat und Saccharosedilaurat, Sorbitanester, wie Sorbitanmonostearat, Polyoxyalkylen-Verbindungen auf Basis von Sorbitanestern, wie Polyoxyethylensorbitanmonostearat.

Das Dispergierhilfsmittel wird üblicherweise im hydrophoben Lösungsmittel gelöst oder dispergiert. Das Dispergierhilfsmittel wird in Mengen zwischen 0.01 und 10 Gew.-%, bevorzugt zwischen 0,2 und 5 Gew.-%, besonders bevorzugt zwischen 0,5 und 2 Gew.-%, bezogen auf die Monomerlösung, eingesetzt. Über Art und Menge des Dispergierhilfsmittels kann der Durchmesser der Monomerlösungstropfen eingestellt werden.

Vorteilhaft werden für die Polymerisation mehrere Rührreaktoren hintereinander geschaltet. Durch die Nachreaktion in weiteren Rührreaktoren kann der Monomerumsatz erhöht und die Rückvermischung vermindert werden. Hierbei ist es weiterhin vorteilhaft, wenn der erste Rührreaktor nicht zu groß ist. Mit steigender Größe des Rührreaktors verbreitert sich zwangsläufig die Größenverteilung der dispergierten Monomerlösungstropfen. Ein kleinerer erster Reaktor ermöglicht daher die Herstellung von Superabsorberpartikeln mit besonders enger Partikelgrößenverteilung.

Die Reaktion wird vorzugsweise unter vermindertem Druck durchgeführt, beispielsweise bei einem Druck von 800 mbar. Über den Druck kann der Siedepunkt der Reaktionsmischung auf die gewünschte Reaktionstemperatur eingestellt werden.

Wird die Polymerisation unter ausreichendem Rückfluss durchgeführt, so kann auf die Inertisierung verzichtet werden. Dabei wird der gelöste Sauerstoff zusammen mit dem verdampfenden Lösungsmittel aus dem Polymerisationsreaktor entfernt.

Die Superabsorberpartikel können in der Polymerdispersion azeotrop entwässert, aus der Polymerdispersion abgetrennt und die abgetrennten Superabsorberpartikel können zur Entfernung des anhaftenden restlichen hydrophoben Lösungsmittels getrocknet werden.

Im Folgenden wird die Oberflächennachvernetzung beschrieben:

Die Polymerpartikel können zur weiteren Verbesserung der Eigenschaften thermisch oberflächennachvernetzt werden. Geeignete Oberflächennachvernetzer sind Verbindungen, die Gruppen enthalten, die mit mindestens zwei Carboxylatgruppen der Polymerpartikel kovalente Bindungen bilden können. Geeignete Verbindungen sind beispielsweise polyfunktionelle Amine, polyfunktionelle Amidoamine, polyfunktionelle Epoxide, wie in EP 0 083 022 A2, EP 0 543 303 A1 und EP 0 937 736 A2 beschrieben, di- oder polyfunktionelle Alkohole, wie in DE 33 14 019 A1, DE 35 23 617 A1 und EP 0 450 922 A2 beschrieben, ß-Hydroxyalkylamide, wie in DE 102 04 938 A1 und US 6,239,230 beschrieben oder Oxazoline wie in EP 0 999 238 A2 beschrieben.

Des Weiteren sind in DE 40 20 780 C1 zyklische Karbonate, in DE 198 07 502 A1 2-Oxazolidinon und dessen Derivate, wie 2-Hydroxyethyl-2-oxazolidinon, in DE 198 07 992 C1 Bis- und Poly-2-oxazolidinone, in DE 198 54 573 A1 2-Oxotetrahydro-1,3-oxazin und dessen Derivate, in DE 198 54 574 A1 N-Acyl-2-Oxazolidinone, in DE 102 04 937 A1 zyklische Harnstoffe, in DE 103 34 584 A1 bizyklische Amidoacetale, in EP 1 199 327 A2 Oxetane und zyklische Harnstoffe, in EP 2 204 388 A1 Oxetane und in WO 03/031482 A1 Morpholin-2,3-dion und dessen Derivate als geeignete Oberflächennachvernetzer beschrieben.

Bevorzugte Oberflächennachvernetzer sind Ethylenkarbonat, Ethylenglykoldiglycidylether, Umsetzungsprodukte von Polyamiden mit Epichlorhydrin und Gemische aus Propylenglykol und 1,4-Butandiol.

Ganz besonders bevorzugte Oberflächennachvernetzer sind 2-Hydroxyethyl-2-oxazolidinon, 2-Oxazolidinon und 1,3-Propandiol.

Weiterhin können auch Oberflächennachvernetzer eingesetzt werden, die zusätzliche polymerisierbare ethylenisch ungesättigte Gruppen enthalten, wie in DE 37 13 601 A1 beschrieben

Die Menge an Oberflächennachvernetzer beträgt vorzugsweise 0,001 bis 3 Gew.-%, besonders bevorzugt 0,02 bis 1 Gew.-%, ganz besonders bevorzugt 0,05 bis 0,2 Gew.-%, jeweils bezogen auf die Polymerpartikel.

Die Oberflächennachvernetzung wird üblicherweise so durchgeführt, dass eine Lösung des Oberflächennachvernetzers auf die getrockneten Polymerpartikel aufgesprüht wird. Im Anschluss an das Aufsprühen werden die mit Oberflächennachvernetzer beschichteten Polymerpartikel oberflächennachvernetzt und getrocknet, wobei die Oberflächennachvernetzungsreaktion sowohl vor als auch während der Trocknung stattfinden kann.

Das Aufsprühen einer Lösung des Oberflächennachvernetzers wird vorzugsweise in Mischern mit bewegten Mischwerkzeugen, wie Schneckenmischer, Scheibenmischer und Schaufelmischer, durchgeführt. Besonders bevorzugt sind Horizontalmischer, wie Schaufelmischer, ganz besonders bevorzugt sind Vertikalmischer. Die Unterscheidung in Horizontalmischer und Vertikalmischer erfolgt über die Lagerung der Mischwelle, d.h. Horizontalmischer haben eine horizontal gelagerte Mischwelle und Vertikalmischer haben eine vertikal gelagerte Mischwelle. Geeignete Mischer sind beispielsweise Horizontale Pflugschar® Mischer (Gebr. Lödige Maschinenbau GmbH; Paderborn; Deutschland), Vrieco-Nauta Continuous Mixer (Hosokawa Micron BV; Doetinchem; Niederlande), Processall Mixmill Mixer (Processall Incorporated; Cincinnati; USA) und Schugi Flexomix® (Hosokawa Micron BV; Doetinchem; Niederlande). Es ist aber auch möglich die Oberflächennachvernetzerlösung in einem Wirbelbett aufzusprühen.

Die Oberflächennachvernetzer werden typischerweise als wässrige Lösung eingesetzt. Über den Gehalt an nichtwässrigem Lösungsmittel bzw. Gesamtlösungsmittelmenge kann die Eindringtiefe des Oberflächennachvernetzers in die Polymerpartikel eingestellt werden.

Wird ausschließlich Wasser als Lösungsmittel verwendet, so wird vorteilhaft ein Tensid zugesetzt. Dadurch wird das Benetzungsverhalten verbessert und die Verklumpungsneigung vermindert. Vorzugsweise werden aber Lösungsmittelgemische eingesetzt, beispielsweise Isopropanol/Wasser, 1,3-Propandiol/Wasser und Propylenglykol/Wasser, 2-Methyl-1,3-Propandiol/Wasser, Ethylenglykol/Wasser, Diethylenglykol/Wasser, Triethylenglykol/Wasser, Tetraethylenglykol/Wasser oder Polyethylenglykol/Wasser, wobei das Mischungsmassenverhältnis vorzugsweise von 20:80 bis 40:60 beträgt.

Die Oberflächennachvernetzung wird vorzugsweise in Kontakttrocknern, besonders bevorzugt Schaufeltrocknern, ganz besonders bevorzugt Scheibentrocknern, durchgeführt. Geeignete Trockner sind beispielsweise Hosokawa Bepex® Horizontal Paddle Dryer (Hosokawa Micron GmbH; Leingarten; Deutschland), Hosokawa Bepex® Disc Dryer (Hosokawa Micron GmbH; Leingarten; Deutschland), Holo-Flite® dryers (Metso Minerals Industries Inc.; Danville; USA) und Nara Paddle Dryer (NARA Machinery Europe; Frechen; Deutschland). Überdies können auch Wirbelschichttrockner eingesetzt werden.

Die Oberflächennachvernetzung kann im Mischer selbst erfolgen, durch Beheizung des Mantels oder Einblasen von Warmluft. Ebenso geeignet ist ein nachgeschalteter Trockner, wie beispielsweise ein Hordentrockner, ein Drehrohrofen oder eine beheizbare Schnecke. Besonders vorteilhaft wird in einem Wirbelschichttrockner gemischt und thermisch oberflächennachvernetzt.

Bevorzugte Reaktionstemperaturen liegen im Bereich 100 bis 250°C, bevorzugt 120 bis 220°C, besonders bevorzugt 130 bis 210°C, ganz besonders bevorzugt 150 bis 200°C. Die bevorzugte Verweilzeit bei dieser Temperatur beträgt vorzugsweise mindestens 10 Minuten, besonders bevorzugt mindestens 20 Minuten, ganz besonders bevorzugt mindestens 30 Minuten, und üblicherweise höchstens 60 Minuten.

In einer bevorzugten Ausführungsform der vorliegenden Erfindung werden die Polymerpartikel nach der Oberflächennachvernetzung gekühlt. Die Kühlung wird vorzugsweise in Kontaktkühlern, besonders bevorzugt Schaufelkühlern, ganz besonders bevorzugt Scheibenkühlern, durchgeführt. Geeignete Kühler sind beispielsweise Hosokawa Bepex® Horizontal Paddle Cooler (Hosokawa Micron GmbH; Leingarten; Deutschland), Hosokawa Bepex® Disc Cooler (Hosokawa Micron GmbH; Leingarten; Deutschland), Holo-Flite® coolers (Metso Minerals Industries Inc.; Danville; USA) und Nara Paddle Cooler (NARA Machinery Europe; Frechen; Deutschland). Überdies können auch Wirbelschichtkühler eingesetzt werden.

Im Kühler werden die Polymerpartikel auf vorzugsweise 40 bis 90°C, besonders bevorzugt 45 bis 80°C, ganz besonders bevorzugt 50 bis 70°C, abgekühlt.

Die Polymerpartikel können zur weiteren Verbesserung der Eigenschaften beschichtet oder nachbefeuchtet werden:

Die Nachbefeuchtung wird vorzugsweise bei 40 bis 120°C, besonders bevorzugt bei 50 bis 110°C, ganz besonders bevorzugt bei 60 bis 100°C, durchgeführt. Bei zu niedrigen Temperaturen neigen die Polymerpartikel zum Verklumpen und bei höheren Temperaturen verdampft bereits merklich Wasser. Die zur Nachbefeuchtung eingesetzte Wassermenge beträgt vorzugsweise von 1 bis 15 Gew.-%, besonders bevorzugt von 2 bis 10 Gew.-%, ganz besonders bevorzugt von 3 bis 8 Gew.-%. Durch die Nachbefeuchtung wird die mechanische Stabilität der Polymerpartikel erhöht und deren Neigung zur statischen Aufladung vermindert.

Geeignete Beschichtungen zur Verbesserung der Quellgeschwindigkeit sowie der Gelbettpermeabilität (GBP) sind beispielsweise anorganische inerte Substanzen, wie wasserunlösliche Metallsalze, organische Polymere, kationische Polymere sowie zwei- oder mehrwertige Metallkationen. Geeignete Beschichtungen zur Staubbindung sind beispielsweise Polyole. Geeignete Beschichtungen gegen die unerwünschte Verbackungsneigung der Polymerpartikel sind beispielsweise pyrogene Kieselsäure, wie Aerosil® 200, und Tenside, wie Span® 20. Geeignete Beschichtungen zur Staubbindung, zur Verringerung der Verbackungsneigung sowie zur Erhöhung der mechanischen Stabilität sind Polymerdispersionen, wie in EP 0 703 265 B1 beschrieben, und Wachse, wie in US 5 840 321 beschrieben.

Methoden:

Die nachfolgend beschriebenen, mit "WSP" bezeichneten Standard-Testmethoden werden beschrieben in: "Standard Test Methods for the Nonwovens Industry", Ausgabe 2005, gemeinsam herausgegeben von den "Worldwide Strategic Partners" EDANA (Avenue Eugene Plasky, 157, 1030 Brüssel, Belgien, www.edana.org) und INDA (1100 Crescent Green, Suite 115, Cary, North Carolina 27518, USA, www.inda.org). Diese Veröffentlichung ist sowohl von EDANA als auch von INDA erhältlich.

Die Messungen sollten, wenn nicht anders angegeben, bei einer Umgebungstemperatur von 23 ± 2 °C und einer relativen Luftfeuchte von 50 ± 10 % durchgeführt werden. Die Superabsorberpartikel werden vor der Messung gut durchmischt.

Restmonomer

Der Gehalt an Restmonomer wird gemäß der von der EDANA empfohlenen Testmethode WSP Nr. 210.2 (05) "Residual Monomers" bestimmt.

Feuchtegehalt

Der Feuchtegehalt wird gemäß der von der EDANA empfohlenen Testmethode Nr. WSP 230.2 (05) "Mass Loss Upon Heating" bestimmt.

Zentrifugenretentionskapazität (Centrifuge Retention Capacity)

Die Zentrifugenretentionskapazität (CRC) wird gemäß der von der EDANA empfohlenen Testmethode Nr. WSP 241.2 (05) "Fluid Retention Capacity in Saline, After Centrifugation" bestimmt.

Absorption unter einem Druck von 21,0 g/cm$^2$ (Absorption under Load)

Die Absorption unter einem Druck von 21,0 g/cm$^2$ (AUL) wird gemäß der von der EDANA empfohlenen Testmethode Nr. WSP 242.2 (05) "Absorption Under Pressure, Gravimetric Determination" bestimmt.

Absorption unter einem Druck von 49,2 g/cm$^2$ (Absorption under High Load)

Die Absorption unter einem Druck von 49,2 g/cm$^2$ (AUHL) wird analog der von der EDANA empfohlenen Testmethode Nr. WSP 242.2 (05) "Absorption Under Pressure, Gravimetric Determination" bestimmt, wobei statt eines Drucks von 21,0 g/cm$^2$ (0.3psi) ein Druck von 49,2 g/cm$^2$ (0.7psi) eingestellt wird.

Extrahierbare

Der Gehalt an extrahierbaren Bestandteilen der wasserabsorbierenden Polymerpartikel wird gemäß der von der EDANA empfohlenen Testmethode Nr. WSP 270.2 (05) "Extractable" bestimmt.

Schüttgewicht

Das Schüttgewicht (ASG) wird gemäß der von der EDANA empfohlenen Testmethode Nr. WSP 260.2 (05) "Density, Gravimetric Determination" bestimmt.

Flüssigkeitsweiterleitung (Saline Flow Conductivity)

Die Flüssigkeitsweiterleitung (SFC) einer gequollenen Gelschicht unter Druckbelastung von 0,3 psi (2070 Pa) wird, wie in EP 0 640 330 A1 beschrieben, als Gel-Layer-Permeability einer gequollenen Gelschicht aus wasserabsorbierenden Polymerpartikeln bestimmt, wobei die in zuvor genannter Patentanmeldung auf Seite 19 und in Figur 8 beschriebene Apparatur dahingehend modifiziert wurde, dass die Glasfritte (40) nicht mehr verwendet wird, der Stempel (39) aus gleichem Kunststoffmaterial besteht wie der Zylinder (37) und jetzt über die gesamte Auflagefläche gleichmäßig verteilt 21 gleichgroße Bohrungen enthält. Die Vorgehensweise sowie Auswertung der Messung bleibt unverändert gegenüber EP 0 640 330 A1. Der Durchfluss wird automatisch erfasst.

Die Flüssigkeitsweiterleitung (SFC) wird wie folgt berechnet:

$$\text{SFC } [\text{cm}^3\text{s/g}] = (Fg(t{=}0) \times L_0)/(d \times A \times WP),$$

wobei $Fg(t{=}0)$ der Durchfluss an NaCl-Lösung in g/s ist, der anhand einer linearen Regressionsanalyse der Daten $Fg(t)$ der Durchflussbestimmungen durch Extrapolation gegen t=0 erhalten wird, $L_0$ die Dicke der Gelschicht in cm, d die Dichte der NaCl-Lösung in $g/cm^3$, A die Fläche der Gelschicht in $cm^2$ und WP der hydrostatische Druck über der Gelschicht in $dyn/cm^2$.

Vortex-Test

In ein 100 ml-Becherglas, welches ein Magnetrührstäbchen der Größe 30 mm x 6 mm enthält, werden 50,0 ml ± 1,0 ml einer 0,9 Gew.-%igen wässrigen Kochsalzlösung gegeben. Mit Hilfe eines Magnetrührers wird die Kochsalzlösung bei 600 Upm gerührt. Es werden dann möglichst schnell 2,000 g ± 0,010 g wasserabsorbierende Polymerpartikel zugegeben, und die Zeit gemessen, die vergeht, bis die Rührtraube durch die Absorption der Kochsalzlösung durch die wasserabsorbierenden Polymerpartikel verschwindet. Dabei kann der ganze Inhalt des Becherglases sich als einheitliche Gelmasse immer noch drehen, aber die Oberfläche der gelierten Kochsalzlösung darf keine individuellen Turbulenzen mehr zeigen. Die benötigte Zeit wird als Vortex berichtet.

mittlere Sphärizität (mSPHT)

Die mittlere Sphärizität (mSPHT) wird mit dem PartAn® 3001 L Partikel Analysator (Microtrac Europe GmbH; Deutschland) bestimmt.

Die zu analysierende Probe wird in einen Trichter eingefüllt. Das rechnergesteuerte Messsystem startet die Dosiervorrichtung und sorgt für einen kontinuierlichen, konzentrationsgeregelten Partikelstrom. Die Partikel fallen vereinzelt durch den Messschacht und erzeugen kontrastreiche Schattenbilder zwischen Lichtquelle und hochauflösender Kamera. Die Lichtquelle wird von der Kamera angesteuert und erzeugt aufgrund sehr kurzer Belichtungszeiten fehlerfreie Bildinformationen für die Mehrfach-Auswertung jedes einzelnen Partikels in Echtzeit.

In einem 3D-Verfahren wird jedes Partikel mehrfach analysiert und das Verfahren liefert so die absoluten Ergebnisse zur Länge, Breite, Dicke, Fläche und Umfang. Über die Anzahl der vom Partikel abgedeckten Pixel wird die Größe und Form berechnet.

mittlere Partikelgröße

Die mittlere Partikelgröße wird mittels der von der EDANA empfohlenen Testmethode Nr. WSP 220.2 (05) "Partikel Size Distribution" ermittelt, wobei die Massenanteile der Siebfraktionen kumuliert aufgetragen werden und die mittlere Partikelgröße graphisch bestimmt wird. Die mittlere Partikelgröße ist hierbei der Wert der Maschenweite, der sich für kumulierte 50 Gew.-% ergibt.

Staubmessung

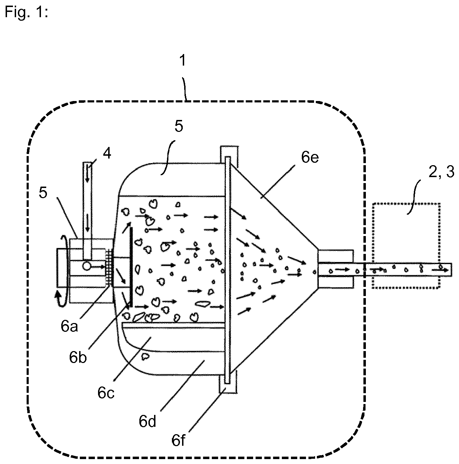

Die Staubmessung wird mit einem Dustmeter (Heubach GmbH, Langelsheim, Deutschland) durchgeführt. Die Figur 1 zeigt den schematischen Aufbau des Dustmeters (1) in Kombination mit einem Aerosolsensor welas® 2070 mit Lichtwellenleiter (2) und einem Streulichtaerosolspektrometersystem Promo® 3000 H (3) (Pelas GmbH, Karlsruhe, Deutschland). Die Zufuhr von trockener Luft (< 10% relative Feuchte) als Transportgas wird über das Regelventil (4) auf 0,1 mbar und einem Durchfluss auf 1,00 $Nm^3$/h eingestellt und über den Luftansaugstutzen (5) in das Dustmeter eingeführt. Zur Messung wurden 25 ± 1 g Superabsorber in einem modifizierten Behälter (6) eingeführt. Der Behälter 6 besteht aus einer Trommel (6a) mit einer Länge von 60 mm und einem Durchmesser von 140 mm, einem Schutzsieb (6b) einer Prallplatte (6c), 3 Mitnehmerblechen in einer Länge von 60 mm und einer Höhe von 22 mm (6d), einem Deckel (6e) und einer Spannvorrichtung (6f). Die Drehzahl des Behälters (6) betrug während der Messung 45 U/min.

Die Anzahl der Staubpartikel wurde mit Hilfe des Streulichtaerosolspektrometersystem Promo® 3000 H in einem Zeitraum von 10 min gezählt und normiert auf 1g Superabsorber pro Minute. Die Staubpartikel wurden in 3 Klassen klassifiziert:

| | |
|---|---|
| Klasse a) | < 1 $\mu$m |
| Klasse b) | 1 - 10 $\mu$m |
| Klasse c) | > 10 $\mu$m |

Beispiele

Beispiel 1

Es wurde ein Superabsorber analog zu Example 1 der WO 2016/134905 A1 hergestellt. Die eingesetzte Monomerlösung enthielt zusätzlich 1,07 Gew.-% des Dinatriumsalzes der 1-Hydro-xyethyliden-1,1-diphosphonsäure.

Die Gaseingangstemperatur der Reaktionszone (5) betrug 167°C, die Gasausgangstemperatur der Reaktionszone (5) betrug 107°C, die Gaseingangstemperatur des internen Wirbelbetts (27) betrug 100°C, die Produkttemperatur im internen Wirbelbett (27) betrug 78°C, die Gasausgangstemperatur der Kondensationskolonne (12) betrug 57°C und die Gasausgangstemperatur der Gastrocknungseinheit (37) betrug 47°C.

Der hergestellte Superabsorber (Grundpolymer) hatte ein Schüttgewicht (ASG) von 0,73 g/ml, eine Zentrifugenretentionskapazität (CRC) von 49,4 g/g, eine Absorption unter einem Druck von 21,0 g/cm$^2$ (AUL) von 10,5 g/g, einen Gehalt an Restmonomer von 5200 ppm, einen Gehalt an Extrahierbaren von 4,5 Gew.-% und einen Feuchtegehalt von 8,0 Gew.-%.

Der Superabsorber wies die folgende Partikelgrößenverteilung auf:

| | |
|---|---|
| >1000 $\mu$m | 0,3 Gew.-% |
| 850 - 1000 $\mu$m | 1,1 Gew.-% |
| 600 - 850 $\mu$m | 3,7 Gew.-% |
| 500 - 600 $\mu$m | 9,9 Gew.-% |
| 400 - 500 $\mu$m | 32,8 Gew.-% |
| 300 - 400 $\mu$m | 40,4 Gew.-% |
| 250 - 300 $\mu$m | 6,4 Gew.-% |
| 200-250 $\mu$m | 4,1 Gew.-% |
| 106 - 200 $\mu$m | 1,2 Gew.-% |
| <106 $\mu$m | 0,1 Gew.-% |

Der Superabsorber wies eine mittlere Partikelgröße (d50) von 377 $\mu$m und eine mittelere Sphärizität (mSPHT) von 0,81 auf.

Das Grundpolymer wurde anschließend analog zu den Beispielen 11 bis 15 der WO 2015/110321 A1 oberflächennachvernetzt. Es wurden 2,0 Gew.-% Ethylenkarbonat, 5,0 Gew.-% Wasser und 0,3 Gew.-% Aluminiumsulfat eingesetzt, jeweils bezogen auf das Grundpolymer. Die Produkttemperatur betrug 160°C und die Höhe des Wehrs betrug 75%.

Im Kühler nach der Oberflächennachvernetzung wurden zunächst 2,35 Gew.-% einer 0,2 gew.- %igen wässrigen Lösung von Sorbitanmonolaurat und anschließend 2,35 Gew.-% einer verdünnten wässrigen Polymerdispersion von Poligen® CE 18 (BASF SE; Ludwigshafen; Deutschland) zugesetzt. Poligen® CE 18 ist eine 21 gew.-%ige wässrige Wachsdispersion eines Ethylen-Acrylsäure-Copolymers aus 20 Gew.-% Acrylsäure und 80 Gew.-% Ethylen, stabilisiert mit Kaliumhydroxid. Das Wachs hat eine Glasübergangstemperatur von 80°C. Die verdünnte wässrige Polymerdispersion wurde so berechnet, dass 500 ppm Wachs als Feststoff, bezogen auf den Superabsorberpartikel auf dem Polymer zugesetzt wurden.

Die Temperatur der Superabsorberpartikel zum Zeitpunkt der Zugabe betrug 75°C.

Der hergestellte oberflächennachvernetze Superabsorber hatte ein Schüttgewicht (ASG) von 0,794 g/ml, eine Zentrifugenretentionskapazität (CRC) von 40,1 g/g, eine Absorption unter einem Druck von 21,0 g/cm$^2$ (AUL) von 32,9 g/g, eine Absorption unter einem Druck von 49,2 g/cm$^2$ (AUHL) von 23,3 g/g, eine Flüssigkeitsweiterleitung (SFC) von

5 x 10$^{-7}$ cm$^3$s/g, einen Vortex von 68 s, einen Feuchtegehalt von 3,2 Gew.-%, einen Gehalt an Restmonomer von 399 ppm und einen Gehalt an Extrahierbaren von 3,2 Gew.-%.

Der oberflächennachvernetzte Superabsorber wies die folgende Partikelgrößenverteilung auf:

| | |
|---|---|
| >850 μm | 0,0 Gew.-% |
| 710 - 850 μm | 0,4 Gew.-% |
| 600 - 710 μm | 2,2 Gew.-% |
| 500 - 600 μm | 9,0 Gew.-% |
| 400 - 500 μm | 36,4 Gew.-% |
| 300 - 400 μm | 39,2 Gew.-% |
| 250 - 300 μm | 7,0 Gew.-% |
| 200 - 250 μm | 4,0 Gew.-% |
| 150 - 200 μm | 1,5 Gew.-% |
| <150 μm | 0,2 Gew.-% |

Der Superabsorber wies eine mittlere Partikelgröße (d50) von 379 μm und eine mittelere Sphärizität (mSPHT) 0,80 auf.

Anschließend wurden die Superabsorberpartikel pneumatisch gefördert.

Als Förderleitung wurde eine glatte Rohrleitung aus Aluminium mit einer Länge von 164 m und einem Innendurchmesser von 100 mm verwendet. Die Förderleitung bestand aus zwei waagerechten und zwei senkrechten Abschnitten, wobei die Abschnitte durch Bögen verbunden waren. Die vertikale Überhöhung betrug insgesamt 13 m. Die Förderleitung hatte einen internen Bypass vom Typ Intraflow (Zepplin Systems GmbH; Friedrichshafen; Deutschland). Die Produktförderung in die Förderleitung erfolgte mittels einer Zellradschleuse CFH250 (Zeppelin Systems GmbH; Friedrichshafen; Deutschland).

Die Förderleistung betrug 7,5 t/h Superabsorberpartikel, die Drehzahl der Zellradschleuse betrug 13,5 U/min, die Förderluftmenge betrug 560 Nm$^3$/h und die Gasgeschwindigkeit betrug 11 m/s am Förderleitungsanfang sowie 11,1 m/s am Förderleitungsende. Der Druck in der Förderleitung betrug von + 660 bis 0 mbar, bezogen auf den Umgebungsdruck. Während der stabilen Förderung lagen die Druckschwankungen bei ± 50 mbar und der durchschnittliche Druck in der Förderung bei 560 mbar. Die Fördergutbeladung betrug 11 kg/kg und die Froude-Zahl am Anfang der Förderung betrug 11.

Nach der Förderung hatte der Superabsorber eine Zentrifugenretentionskapazität (CRC) von 39,5 g/g, eine Absorption unter einem Druck von 21,0 g/cm$^2$ (AUL) von 31,1 g/g, eine Absorption unter einem Druck von 49,2 g/cm$^2$ (AUHL) von 23,1 g/g, eine Flüssigkeitsweiterleitung (SFC) von 4 x 10$^{-7}$ cm$^3$s/g.

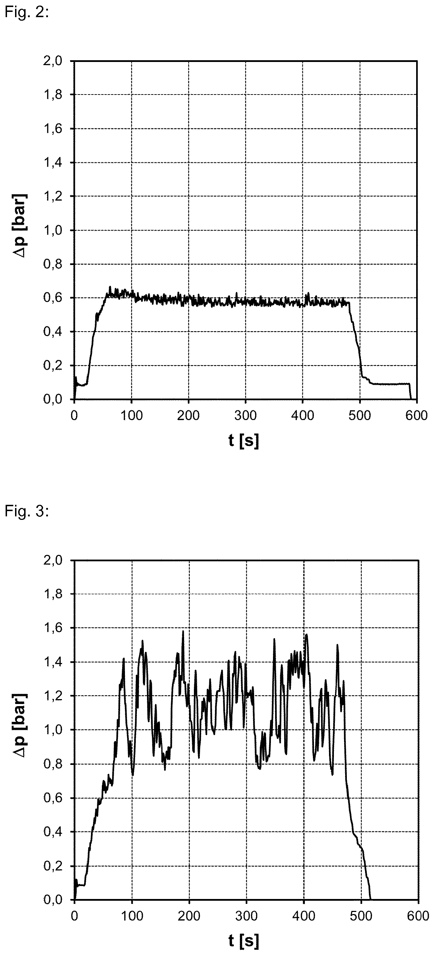

Den Anfangsdruck in der Förderleitung in Abhängigkeit von der Zeit zeigt Figur 2.

Die Anzahl der Staubpartikel von Beispiel 1 vor und nach der pneumatischen Förderung ist in Tabelle 1 zusammengefasst.

Beispiel 2 (Vergleichsbeispiel)

Es wurde verfahren wie unter Beispiel 1. Die zugesetzte Menge an Poligen® CE 18 wurde auf 125 ppm, bezogen auf die Superabsorberpartikel, gesenkt.

Der hergestellte oberflächennachvernetzte Superabsorber hatte ein Schüttgewicht (ASG) von 0,761 g/ml, eine Zentrifugenretentionskapazität (CRC) von 39,3 g/g, eine Absorption unter einem Druck von 21,0 g/cm$^2$ (AUL) von 32,7 g/g, eine Absorption unter einem Druck von 49,2 g/cm$^2$ (AUHL) von 23,1 g/g, eine Flüssigkeitsweiterleitung (SFC) von 5 x 10$^{-7}$ cm$^3$s/g, einen Vortex von 65 s, einen Feuchtegehalt von 3,1 Gew.-%, einen Gehalt an Restmonomer von 428 ppm und einen Gehalt an Extrahierbaren von 3,0 Gew.-%.

Der oberflächennachvernetzte Superabsorber wies die folgende Partikelgrößenverteilung auf:

| | |
|---|---|
| >850 μm | 0,0 Gew.-% |
| 710 - 850 μm | 0,4 Gew.-% |
| 600 - 710 μm | 2,9 Gew.-% |
| 500 - 600 μm | 8,6 Gew.-% |
| 400 - 500 μm | 36,4 Gew.-% |
| 300 - 400 μm | 39,5 Gew.-% |
| 250 - 300 μm | 6,8 Gew.-% |

(fortgesetzt)

| | |
|---|---|
| 200 - 250 $\mu$m | 3,8 Gew.-% |
| 150 - 200 $\mu$m | 1,1 Gew.-% |
| <150 $\mu$m | 0,3 Gew.-% |

Der Superabsorber wies eine mittlere Partikelgröße (d50) von 380 $\mu$m und eine mittelere Sphärizität (mSPHT) 0,80 auf.

Die Förderleistung betrug 7,6 t/h Superabsorberpartikel, die Drehzahl der Zellradschleuse betrug 13,5 U/min, die Förderluftmenge betrug 560 Nm$^3$/h und die Gasgeschwindigkeit betrug nun 7,7 m/s am Förderleitungsanfang sowie 16,4 m/s am Förderleitungsende. Der Druck in der Förderleitung betrug von + 1600 bis 0 mbar, bezogen auf den Umgebungsdruck. Die Fördergutbeladung betrug 12 kg/kg und die Froude-Zahl am Anfang der Förderung betrug 7,7.

Ein gleichmäßiger Betrieb der pneumatischen Förderung mit einer Förderluftmenge von 560 Nm$^3$/h war nicht möglich. Während der instabilen Förderung lagen die Druckschwankungen bei $\pm$ 450 mbar und der durchschnittliche Druck in der Förderung bei 1120 mbar.

Nach der Förderung hatte der Superabsorber eine Zentrifugenretentionskapazität (CRC) von 39,3 g/g, eine Absorption unter einem Druck von 21,0 g/cm$^2$ (AUL) von 30,7 g/g, eine Absorption unter einem Druck von 49,2 g/cm$^2$ (AUHL) von 21,6 g/g, eine Flüssigkeitsweiterleitung (SFC) von 3 x 10$^{-7}$ cm$^3$s/g.

Den Anfangsdruck in der Förderleitung in Abhängigkeit von der Zeit zeigt Figur 3.

Die Anzahl der Staubpartikel von Beispiel 2 vor und nach der pneumatischen Förderung ist in Tabelle 1 zusammengefasst.

Beispiel 3 (Vergleichsbeispiel)

Es wurde verfahren wie unter Beispiel 1. Es wurde kein Poligen® CE 18 zugesetzt.

Der hergestellte oberflächennachvernetze Superabsorber hatte ein Schüttgewicht (ASG) von 0,78 g/ml, eine Zentrifugenretentionskapazität (CRC) von 39,6 g/g, eine Absorption unter einem Druck von 21,0 g/cm$^2$ (AUL) von 33,2 g/g, eine Absorption unter einem Druck von 49,2 g/cm$^2$ (AUHL) von 24,8 g/g, eine Flüssigkeitsweiterleitung (SFC) von 5 x 10$^{-7}$ cm$^3$s/g, einen Vortex von 66 s, einen Feuchtegehalt von 3,5 Gew.-%, einen Gehalt an Restmonomer von 386 ppm und einen Gehalt an Extrahierbaren von 3,0 Gew.-%.

Der oberflächennachvernetzte Superabsorber wies die folgende Partikelgrößenverteilung auf:

| | |
|---|---|
| >850 $\mu$m | 0,0 Gew.-% |
| 710 - 850 $\mu$m | 0,4 Gew.-% |
| 600 - 710 $\mu$m | 2,3 Gew.-% |
| 500 - 600 $\mu$m | 10,0 Gew.-% |
| 400 - 500 $\mu$m | 36,3 Gew.-% |
| 300 - 400 $\mu$m | 39,4 Gew.-% |
| 250 - 300 $\mu$m | 6,0 Gew.-% |
| 200-250 $\mu$m | 4,1 Gew.-% |
| 150 - 200 $\mu$m | 1,3 Gew.-% |
| <150 $\mu$m | 0,2 Gew.-% |

Der Superabsorber wies eine mittlere Partikelgröße (d50) von 383 $\mu$m und eine mittelere Sphärizität (mSPHT) 0,79 auf.

Die Förderleistung betrug 7,2 t/h Superabsorberpartikel, die Drehzahl der Zellradschleuse betrug 13,5 U/min, die Förderluftmenge betrug 560 Nm$^3$/h und die Gasgeschwindigkeit betrug nun 7,5 m/s am Förderleitungsanfang sowie 16,4 m/s am Förderleitungsende. Der Druck in der Förderleitung betrug von + 2400 bis 0 mbar, bezogen auf den Umgebungsdruck. Während der Förderung lagen die Druckschwankungen bei $\pm$ 1000 mbar. Die Fördergutbeladung betrug 11,3 kg/kg und die Froude-Zahl am Anfang der Förderung betrug 7,5. Die Druckspitzen während der Förderung war durch starkes Schlagen in der Leitung deutlich zu hören.

Ein gleichmäßiger Betrieb der pneumatischen Förderung mit einer Förderluftmenge von 560 Nm$^3$/h war nicht möglich. Während der sehr instabilen Förderung lagen die Druckschwankungen bei $\pm$ 900 mbar und der durchschnittliche Druck in der Förderung bei 1160 mbar.

Nach der Förderung hatte der Superabsorber eine Zentrifugenretentionskapazität (CRC) von 38,6 g/g, eine Absorption unter einem Druck von 21,0 g/cm$^2$ (AUL) von 32,0 g/g, eine Absorption unter einem Druck von 49,2 g/cm$^2$

(AUHL) von 20,6 g/g, eine Flüssigkeitsweiterleitung (SFC) von 2 x $10^{-7}$ cm$^3$s/g.

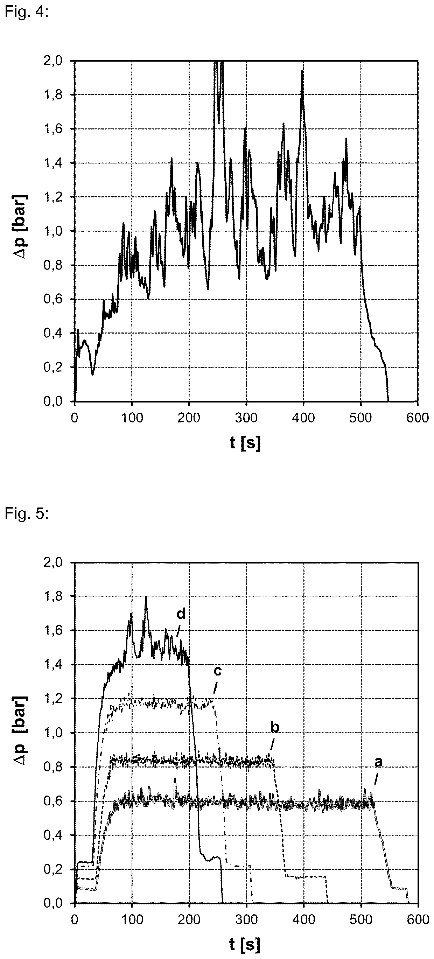

Den Anfangsdruck in der Förderleitung in Abhängigkeit von der Zeit zeigt Figur 4.

Die Anzahl der Staubpartikel von Beispiel 3 vor und nach der pneumatischen Förderung ist in Tabelle 1 zusammengefasst.

Beispiel 4

Das Grundpolymer aus Beispiel 1 wurde anschließend analog zu den Beispielen 11 bis 15 der WO 2015/110321 A1 oberflächennachvernetzt. Es wurden 2,0 Gew.-% Ethylenkarbonat, 5,0 Gew.-% Wasser und 0,05 Gew.-% Aluminiumsulfat eingesetzt, jeweils bezogen auf das Grundpolymer. Die Produkttemperatur betrug 159°C und die Höhe des Wehrs betrug 75%.

Im Kühler nach der Oberflächennachvernetzung wurden zunächst 4,35 Gew.-% einer 0,23 gew.-%igen wässrigen Lösung von Aluminiumlaktat und anschließend 1,66 Gew.-% einer verdünnten wässrigen Polymerdispersion von Poligen® CE 18 (BASF SE; Ludwigshafen; Deutschland) und Sorbitanmonolaurat zugesetzt. Die verdünnte wässrige Polymerdispersion wurde so berechnet, dass 500 ppm Wachs als Feststoff und 25 ppm Sorbitanmonolaurat, bezogen auf die Superabsorberpartikel, zugesetzt wurden.

Der hergestellte oberflächennachvernetzte Superabsorber hatte ein Schüttgewicht (ASG) von 0,75 g/ml, eine Zentrifugenretentionskapazität (CRC) von 38,1g/g, eine Absorption unter einem Druck von 21,0 g/cm$^2$ (AUL) von 34,4 g/g, eine Absorption unter einem Druck von 49,2 g/cm$^2$ (AUHL) von 25,9 g/g, eine Flüssigkeitsweiterleitung (SFC) von 5 x $10^{-7}$ cm$^3$s/g, einen Vortex von 69 s, einen Feuchtegehalt von 4,5 Gew.-%, einen Gehalt an Restmonomer von 263 ppm und einen Gehalt an Extrahierbaren von 2,6 Gew.-%.

Der oberflächennachvernetzte Superabsorber wies die folgende Partikelgrößenverteilung auf:

| | |
|---|---|
| >850 $\mu$m | 0,0 Gew.-% |
| 710 - 850 $\mu$m | 0,1 Gew.-% |
| 600 - 710 $\mu$m | 2,5 Gew.-% |
| 500 - 600 $\mu$m | 12,5 Gew.-% |
| 400 - 500 $\mu$m | 38,2 Gew.-% |
| 300 - 400 $\mu$m | 37,4 Gew.-% |
| 250 - 300 $\mu$m | 5,0 Gew.-% |
| 200 - 250 $\mu$m | 3,0 Gew.-% |
| 150 - 200 $\mu$m | 0,6 Gew.-% |
| <150 $\mu$m | 0,2 Gew.-% |

Der Superabsorber wies eine mittlere Partikelgröße (d50) von 396 $\mu$m und eine mittelere Sphärizität (mSPHT) 0,80 auf.

Die so erhaltenen Superabsorberpartikel wurden unter unterschiedlichen Bedingungen pneumatisch gefördert (Beispiele 4a bis 4d).

Beispiel 4 a

Die Förderleistung bei der ersten Förderung betrug 7,2 t/h Superabsorberpartikel, die Drehzahl der Zellradschleuse betrug 13,5 U/min, die Förderluftmenge betrug 550 Nm$^3$/h und die Gasgeschwindigkeit betrug nun 10,7 m/s am Förderleitungsanfang sowie 17,3 m/s am Förderleitungsende. Der Druck in der Förderleitung betrug von + 740 bis 0 mbar, bezogen auf den Umgebungsdruck. Die Fördergutbeladung betrug 10,9 kg/kg und die Froude-Zahl am Anfang der Förderung betrug 11. Während der stabilen Förderung lagen die Druckschwankungen bei $\pm$ 50 mbar und der durchschnittliche Druck in der Förderung bei 580 mbar.

Nach der Förderung hatte der Superabsorber eine Zentrifugenretentionskapazität (CRC) von 38,7 g/g, eine Absorption unter einem Druck von 21,0 g/cm$^2$ (AUL) von 34,2 g/g, eine Absorption unter einem Druck von 49,2 g/cm$^2$ (AUHL) von 26,2 g/g, eine Flüssigkeitsweiterleitung (SFC) von 5 x $10^{-7}$ cm$^3$s/g.

Die Anzahl der Staubpartikel von Beispiel 4a vor und nach der pneumatischen Förderung ist in Tabelle 1 zusammengefasst.

Beispiel 4 b

Die Förderleistung bei der anschließenden zweiten Förderung betrug 11,2 t/h Superabsorberpartikel, die Drehzahl der Zellradschleuse betrug 20 U/min, die Förderluftmenge betrug 660 Nm$^3$/h und die Gasgeschwindigkeit betrug nun 11,0 m/s am Förderleitungsanfang sowie 20,4 m/s am Förderleitungsende. Der Druck in der Förderleitung betrug von + 910 bis 0 mbar, bezogen auf den Umgebungsdruck. Während der Förderung lagen die Druckschwankungen bei ± 50 mbar. Die Fördergutbeladung betrug 14,2 kg/kg und die Froude-Zahl am Anfang der Förderung betrug 11. Während der stabilen Förderung lagen die Druckschwankungen bei ± 50 mbar und der durchschnittliche Druck in der Förderung bei 825 mbar.

Nach der Förderung hatte der Superabsorber eine Zentrifugenretentionskapazität (CRC) von 38,6 g/g, eine Absorption unter einem Druck von 21,0 g/cm$^2$ (AUL) von 33,6 g/g, eine Absorption unter einem Druck von 49,2 g/cm$^2$ (AUHL) von 25,3 g/g, eine Flüssigkeitsweiterleitung (SFC) von 4 x 10$^{-7}$ cm$^3$s/g.

Beispiel 4 c

Die Förderleistung bei der anschließenden dritten Förderung betrug 16,4 t/h Superabsorberpartikel, die Drehzahl der Zellradschleuse betrug 30 U/min, die Förderluftmenge betrug 560 Nm$^3$/h und die Gasgeschwindigkeit betrug nun 10,6 m/s am Förderleitungsanfang sowie 23,1 m/s am Förderleitungsende. Der Druck in der Förderleitung betrug von + 1230 bis 0 mbar, bezogen auf den Umgebungsdruck. Während der Förderung lagen die Druckschwankungen bei ± 50 mbar. Die Fördergutbeladung betrug 18,3 kg/kg und die Froude-Zahl am Anfang der Förderung betrug 10. Während der stabilen Förderung lagen die Druckschwankungen bei ± 50 mbar und der durchschnittliche Druck in der Förderung bei 1160 mbar.

Nach der Förderung hatte der Superabsorber eine Zentrifugenretentionskapazität (CRC) von 38,6 g/g, eine Absorption unter einem Druck von 21,0 g/cm$^2$ (AUL) von 33,0 g/g, eine Absorption unter einem Druck von 49,2 g/cm$^2$ (AUHL) von 24,2 g/g, eine Flüssigkeitsweiterleitung (SFC) von 4 x 10$^{-7}$ cm$^3$s/g.

Beispiel 4 d

Die Förderleistung bei der anschließenden vierten Förderung betrug 20,8 t/h Superabsorberpartikel, die Drehzahl der Zellradschleuse betrug 40 U/min, die Förderluftmenge betrug 770 Nm$^3$/h und die Gasgeschwindigkeit betrug nun 9,1 m/s am Förderleitungsanfang sowie 23,2 m/s am Förderleitungsende. Der Druck in der Förderleitung betrug von + 1800 bis 0 mbar, bezogen auf den Umgebungsdruck. Während der Förderung lagen die Druckschwankungen bei ± 50 mbar. Die Fördergutbeladung betrug 23,1 kg/kg und die Froude-Zahl am Anfang der Förderung betrug 9. Während der stabilen Förderung lagen die Druckschwankungen bei ± 200 mbar und der durchschnittliche Druck in der Förderung bei 1500 mbar.

Nach der Förderung hatte der Superabsorber eine Zentrifugenretentionskapazität (CRC) von 38,6 g/g, eine Absorption unter einem Druck von 21,0 g/cm$^2$ (AUL) von 32,8 g/g, eine Absorption unter einem Druck von 49,2 g/cm$^2$ (AUHL) von 23,9 g/g, eine Flüssigkeitsweiterleitung (SFC) von 3 x 10$^{-7}$ cm$^3$s/g.

Den Anfangsdruck in der Förderleitung in Abhängigkeit von der Zeit der Beispiele 4a bis 4d zeigt Figur 5.

Tab. 1: Aus dem Heubach-Dustmeter in 5l/min trockener Luft ausgetragene normierte Partikelanzahl P, normiert pro min und pro g Superabsorber, klassifiziert und als Summe

| Beispiel | Probenahme | normierter Partikelanzahl [P/min/g] | | | |
|---|---|---|---|---|---|
| | | < 1 $\mu$m | 1$\mu$m - 10$\mu$m | > 10$\mu$m | Summe |
| 1 | vor Förderung | 60154 | 118915 | 3189 | 182258 |
| | nach Förderung | 81525 | 151647 | 4232 | 237404 |
| 2*) | vor Förderung | 126543 | 197421 | 3068 | 327032 |
| | nach Förderung | 266433 | 353877 | 6027 | 626337 |
| 3*) | vor Förderung | 419557 | 442039 | 3511 | 865107 |
| | nach Förderung | 682969 | 602259 | 4377 | 1289605 |
| 4a | vor Förderung | 27042 | 71293 | 2231 | 100566 |
| | nach Förderung | 52688 | 114416 | 5455 | 172559 |
| *) Vergleichsbeispiel | | | | | |

**Patentansprüche**

1. Verfahren zur pneumatischen Förderung von Superabsorberpartikeln, **dadurch gekennzeichnet, dass** die Superabsorberpartikel vor der pneumatischen Förderung mit einer wässrigen Wachsdispersion versetzt wurden, das Wachs eine Glasübergangstemperatur von mindestens 65°C aufweist und bezogen auf die unbehandelten Superabsorberpartikel von 0,020 bis 0,20 Gew.-% Wachs eingesetzt wurden.

2. Verfahren nach Anspruch 1, **dadurch gekennzeichnet, dass** das Wachs eine Glasübergangstemperatur von mindestens 80°C aufweist.

3. Verfahren nach Anspruch 1 oder 2, **dadurch gekennzeichnet, dass** bezogen auf die Superabsorberpartikel von 0,035 bis 0,08 Gew.-% Wachs eingesetzt wurden.

4. Verfahren nach einem der Ansprüche 1 bis 3, **dadurch gekennzeichnet, dass** das Wachs ein Copolymer aus 70 bis 95 mol-% mindestens einem ethylenisch ungesättigten Kohlenwasserstoff und 5 bis 30 mol-% mindestens einer ethylenisch ungesättigten Carbonsäure ist.

5. Verfahren nach einem der Ansprüche 1 bis 4, **dadurch gekennzeichnet, dass** die Superabsorberpartikel eine mittlere Partikelgröße von 250 bis 500 μm aufweisen.

6. Verfahren nach einem der Ansprüche 1 bis 5, **dadurch gekennzeichnet, dass** die Superabsorberpartikel eine mittlere Sphärizität (mSPHT) von größer 0,72 aufweisen.

7. Verfahren nach einem der Ansprüche 1 bis 6, **dadurch gekennzeichnet, dass** die Temperatur der Superabsorberpartikel während der pneumatischen Förderung mindestens 40°C und von mindestens 20°C weniger als der Glasübergangstemperatur des Wachses beträgt.

8. Verfahren nach einem der Ansprüche 1 bis 7, **dadurch gekennzeichnet, dass** die Gasanfangsgeschwindigkeit bei der pneumatischen Förderung einer Froude-Zahl von 2 bis 40 entspricht.

9. Verfahren nach einem der Ansprüche 1 bis 8, **dadurch gekennzeichnet, dass** die Fördergutbeladung bei der pneumatischen Förderung von 1 bis 30 kg/kg beträgt und die Fördergutbeladung der Quotient aus Fördergutmassenstrom und Gasmassenstrom ist.

10. Zusammensetzung, enthaltend Superabsorberpartikel und Wachspartikel, wobei sich die Wachspartikel auf der Oberfläche der Superabsorberpartikel befinden, die Wachspartikel in Wasser dispergierbar sind und eine Glasübergangstemperatur von mindestens 65°C aufweisen, der Anteil der Wachspartikel bezogen auf die Superabsorberpartikel von 0,02 bis 0,2 Gew.-% beträgt und die Zusammensetzung eine Absorption unter einem Druck von 4,83 kPa (AUL0.7psi) von mindestens 10 g/g aufweist.

11. Zusammensetzung nach Anspruch 10, wobei das Wachs eine Glasübergangstemperatur von mindestens 80°C aufweist.

12. Zusammensetzung nach Anspruch 10 oder 11, wobei bezogen auf die Superabsorberpartikel von 0,035 bis 0,08 Gew.-% Wachs eingesetzt wurden.

13. Zusammensetzung nach einem der Ansprüche 10 bis 12, wobei das Wachs ein Copolymer aus 5 bis 30 mol-% mindestens einer ethylenisch ungesättigten Carbonsäure und 70 bis 95 mol-% mindestens einem ethylenisch ungesättigten Kohlenwasserstoff ist.

14. Zusammensetzung nach einem der Ansprüche 10 bis 13, wobei die Superabsorberpartikel eine mittlere Partikelgröße von 250 bis 500 μm aufweisen.

15. Zusammensetzung nach einem der Ansprüche 10 bis 14, wobei die Superabsorberpartikel eine mittlere Sphärizität (mSPHT) von größer 0,72 aufweisen.

## Claims

1. A method of pneumatically conveying superabsorbent particles, wherein the superabsorbent particles have been admixed with an aqueous wax dispersion prior to the pneumatic conveying, the wax has a glass transition temperature of at least 65°C and, based on the untreated superabsorbent particles, from 0.020% to 0.20% by weight of wax has been used.

2. The method according to claim 1, wherein the wax has a glass transition temperature of at least 80°C.

3. The method according to claim 1 or 2, wherein, based on the superabsorbent particles, from 0.035% to 0.08% by weight of wax has been used.

4. The method according to any of claims 1 to 3, wherein the wax is a copolymer of 70 to 95 mol% of at least one ethylenically unsaturated hydrocarbon and 5 to 30 mol% of at least one ethylenically unsaturated carboxylic acid.

5. The method according to any of claims 1 to 4, wherein the superabsorbent particles have an average particle size of 250 to 500 $\mu$m.

6. The method according to any of claims 1 to 5, wherein the superabsorbent particles have an average sphericity (ASPHT) of greater than 0.72.

7. The method according to any of claims 1 to 6, wherein the temperature of the superabsorbent particles during the pneumatic conveying is at least 40°C, and at least 20°C less than the glass transition temperature of the wax.

8. The method according to any of claims 1 to 7, wherein the initial gas velocity in the pneumatic conveying corresponds to a Froude number of 2 to 40.

9. The method according to any of claims 1 to 8, wherein the conveying material load in the pneumatic conveying is from 1 to 30 kg/kg and the conveying material load is the quotient of conveying material mass flow rate and gas mass flow rate.

10. A composition, comprising superabsorbent particles and wax particles, wherein the wax particles are on the surface of the superabsorbent particles, the wax particles are dispersible in water and have a glass transition temperature of at least 65°C, the proportion of the wax particles based on the superabsorbent particles is from 0.02% to 0.2% by weight, and the composition has an absorption under a pressure of 4.83 kPa (AUL0.7psi) of at least 10 g/g.

11. The composition according to claim 10, wherein the wax has a glass transition temperature of at least 80°C.

12. The composition according to claim 10 or 11, wherein, based on the superabsorbent particles, from 0.035% to 0.08% by weight of wax has been used.

13. The composition according to any of claims 10 to 12, wherein the wax is a copolymer of 5 to 30 mol% of at least one ethylenically unsaturated carboxylic acid and 70 to 95 mol% of at least one ethylenically unsaturated hydrocarbon.

14. The composition according to any of claims 10 to 13, wherein the superabsorbent particles have an average particle size of 250 to 500 $\mu$m.

15. The composition according to any of claims 10 to 14, wherein the superabsorbent particles have an average sphericity (ASPHT) of greater than 0.72.

## Revendications

1. Procédé pour le transport pneumatique de particules de superabsorbant, **caractérisé en ce que** les particules de superabsorbant ont été mélangées avec une dispersion aqueuse de cire avant le transport pneumatique, la cire présente une température de transition vitreuse d'au moins 65 °C et de 0,020 à 0,20 % en poids de cire ont été utilisés, par rapport aux particules de superabsorbant non traitées.

**2.** Procédé selon la revendication 1, **caractérisé en ce que** la cire présente une température de transition vitreuse d'au moins 80 °C.

**3.** Procédé selon la revendication 1 ou 2, **caractérisé en ce que** de 0,035 à 0, 08 % en poids de cire ont été utilisés, par rapport aux particules de superabsorbant.

**4.** Procédé selon l'une quelconque des revendications 1 à 3, **caractérisé en ce que** la cire est un copolymère composé de 70 à 95 % en moles d'au moins un hydrocarbure éthyléniquement insaturé et 5 à 30 % en moles d'au moins un acide carboxylique éthyléniquement insaturé.

**5.** Procédé selon l'une quelconque des revendications 1 à 4, **caractérisé en ce que** les particules de superabsorbant présentent une taille moyenne de particule de 250 à 500 μm.

**6.** Procédé selon l'une quelconque des revendications 1 à 5, **caractérisé en ce que** les particules de superabsorbant présentent une sphéricité moyenne (mSPHT) supérieure à 0,72.

**7.** Procédé selon l'une quelconque des revendications 1 à 6, **caractérisé en ce que** la température des particules de superabsorbant pendant le transport pneumatique est d'au moins 40 °C et d'au moins 20 °C plus basse que la température de transition vitreuse de la cire.

**8.** Procédé selon l'une quelconque des revendications 1 à 7, **caractérisé en ce que** la vitesse initiale de gaz lors du transport pneumatique correspond à un nombre de Froude de 2 à 40.

**9.** Procédé selon l'une quelconque des revendications 1 à 8, **caractérisé en ce que** la charge de matière transportée lors du transport pneumatique est de 1 à 30 kg/kg et la charge de matière transportée étant le quotient du flux de masse de matière transportée et du flux de masse de gaz.

**10.** Composition, contenant des particules de superabsorbant et des particules de cire, les particules de cire se trouvant sur la surface des particules de superabsorbant, les particules de cire étant dispersibles dans l'eau et présentant une température de transition vitreuse d'au moins 65 °C, la proportion des particules de cire par rapport aux particules de superabsorbant étant de 0,02 à 0,2 % en poids et la composition présentant une absorption sous une pression de 4,83 kPa (AUL 0,7 psi) d'au moins 10 g/g.

**11.** Composition selon la revendication 10, la cire présentant une température de transition vitreuse d'au moins 80 °C.

**12.** Composition selon la revendication 10 ou 11, de 0,035 à 0,08 % en poids de cire ayant été utilisés, par rapport aux particules de superabsorbant.

**13.** Composition selon l'une quelconque des revendications 10 à 12, la cire étant un copolymère composé de 5 à 30 % en moles d'au moins un acide carboxylique éthyléniquement insaturé et 70 à 95 % en moles d'au moins un hydrocarbure éthyléniquement insaturé.

**14.** Composition selon l'une quelconque des revendications 10 à 13, les particules de superabsorbant présentant une taille moyenne de particule de 250 à 500 μm.

**15.** Composition selon l'une quelconque des revendications 10 à 14, les particules de superabsorbant présentant une sphéricité moyenne (mSPHT) supérieure à 0,72.

Fig. 1:

Fig. 2:

Fig. 3:

Fig. 4:

Fig. 5:

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0755964 A2
- WO 2008077779 A1
- US 2017361305 A
- WO 200031157 A1
- WO 9955767 A1
- EP 0530438 A1
- EP 0547847 A1
- EP 0559476 A1
- EP 0632068 A1
- WO 9321237 A1
- WO 03104299 A1
- WO 03104300 A1
- WO 03104301 A1
- DE 10331450 A1
- DE 10331456 A1
- DE 10355401 A1
- DE 19543368 A1
- DE 19646484 A1
- WO 9015830 A1
- WO 02032962 A2
- WO 2017170604 A1
- WO 2001038402 A1
- DE 3825366 A1
- US 6241928 B
- WO 2014079694 A1
- WO 2015110321 A1

- WO 2008068208 A1
- WO 2015062883 A2
- EP 0083022 A2
- EP 0543303 A1
- EP 0937736 A2
- DE 3314019 A1
- DE 3523617 A1
- EP 0450922 A2
- DE 10204938 A1
- US 6239230 B
- EP 0999238 A2
- DE 4020780 C1
- DE 19807502 A1
- DE 19807992 C1
- DE 19854573 A1
- DE 19854574 A1
- DE 10204937 A1
- DE 10334584 A1
- EP 1199327 A2
- EP 2204388 A1
- WO 03031482 A1
- DE 3713601 A1
- EP 0703265 B1
- US 5840321 A
- EP 0640330 A1
- WO 2016134905 A1

**In der Beschreibung aufgeführte Nicht-Patentliteratur**

- Modern Superabsorbent Polymer Technology. **F.L. BUCHHOLZ ; A.T. GRAHAM.** Monographie. Wiley-VCH, 1998, 71-103
- **G. KANIG.** *Kolloid-Zeitschrift & Zeitschrift für Polymere,* vol. 190, 1
- Ullmann's Ecyclopedia of Industrial Chemistry. VCH Weinheim, 1992, vol. A21, 169

- **J. BRANDRUP ; E.H. IMMERGUT.** Polymer Handbook. J. Wiley, 1996
- POLYMER HANDBOOK. J.Wiley, 1975
- POLYMER HANDBOOK. J. Wiley, 1989
- **T.G. FOX.** *Bull. Amer. Phys. Soc.,* 1956, vol. 1, 123
- Standard Test Methods for the Nonwovens Industry. 2005